# EUROPÄISCHE PATENTSCHRIFT

(11) **EP 3 050 870 B1**
(45) Veröffentlichungstag und Bekanntmachung des Hinweises auf die Patenterteilung: **27.11.2024**
(21) Anmeldenummer: 15153287.6
(22) Anmeldetag: 30.01.2015
(51) Int. Cl.: C07C 209/48, C07C 209/52, B01J 25/00, B01J 35/40, C07C 211/36

(54) **Verfahren zur Herstellung von 3-Aminomethyl-3,5,5-trimethylcyclohexylamin**
Method for the preparation of 3-aminomethyl-3,5,5-trimethylcyclohexylamine
Procédé de fabrication de 3-aminométhyl-3,5,5-triméthylcyclohexylamine

(43) Veröffentlichungstag der Anmeldung: 03.08.2016
(73) Patentinhaber: Evonik Operations GmbH, 45128 Essen (DE)
(72) Erfinder: Kohlstruk, Stephan, 45966 Gladbeck (DE); Rittsteiger, Anne, 59399 Olfen (DE); Rüfer, Alexander Martin, 45657 Recklinghausen (DE); Schlueter, Norbert, 48712 Gescher (DE); Schneider, Sven, 45711 Datteln (DE); Sowka, Sabrina, 48249 Dülmen (DE); Streukens, Guido, 42111 Wuppertal (DE); Röder, Stefan, 36391 Sinntal (DE); Berweiler, Monika, 63477 Maintal (DE)
(74) Vertreter: Evonik Patent Association

(56) Entgegenhaltungen:
- WO-A1-2005/039764
- WO-A1-2007/028411
- WO-A1-2008/107226
- JP-A- H07 188 126
- JP-A- S62 123 154

## Beschreibung

Die Erfindung betrifft ein verbessertes Verfahren zur Herstellung von 3-Aminomethyl-3,5,5-trimethylcyclohexylamin, nachfolgend Isophorondiamin oder abgekürzt IPDA genannt, mittels katalytischer Hydrierung und/oder katalytischer reduktiver Aminierung (auch als aminierende Hydrierung bezeichnet) von 3-Cyano-3,5,5-trimethylcyclohexanon, nachfolgend Isophoronnitril oder abgekürzt IPN genannt.

Die Herstellung von IPDA durch aminierende Hydrierung von IPN ist bekannt und bereits mehrfach beschrieben worden.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

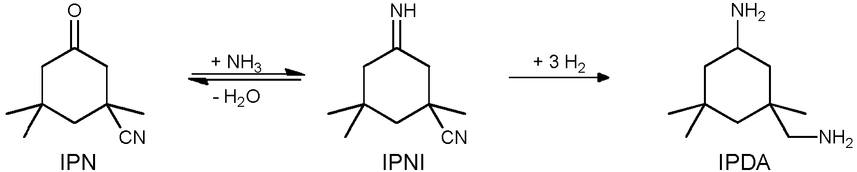

Die Ausbeute an IPDA wird bei dieser Art der Reaktionsführung maßgeblich durch den Überschuss an Ammoniak bestimmt. Die maximal erzielten IPDA-Ausbeuten liegen bei ca. 80 %. Hauptnebenprodukt ist der sogenannte Aminoalkohol, IPAA, der aus der direkten Hydrierung des IPN resultiert.

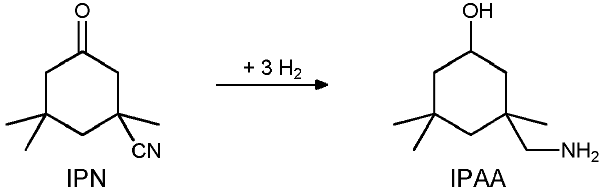

Eine wesentliche Steigerung der IPDA-Ausbeute wird erreicht, wenn die Bildung des IPNI durch Einsatz geeigneter Iminierungskatalysatoren beschleunigt wird. Als Iminierungskatalysatoren sind z. B. saure lonenaustauscherharze (EP 042 119) geeignet. Daneben können auch acide Metalloxide (EP 449 089), sulfonsäuregruppenhaltige Organopolysiloxane (EP 816 323), Heteropolysäuren (DE 44 26 472) und Aktivkohle (EP 061 137) als Iminierungskatalysatoren eingesetzt werden. Neben der Reduzierung des unerwünschten Aminoalkohols werden auch andere Nebenprodukte deutlich zurück gedrängt, z. B. bicyclische Verbindungen und solche Nebenprodukte, die aus der Abspaltung von HCN resultieren.

Auf die Problematik der Abspaltung von HCN aus gamma-Ketonitrilen, wie dem IPN, wird in der Literatur besonders hingewiesen (US 3,352,913). Zum Einen wird bemerkt, dass durch die HCN-Abspaltung die Ausbeute an IPDA reduziert wird (EP 042 119, DE 44 26 472).

Zum Anderen wird darauf hingewiesen, dass HCN als Katalysatorgift wirkt und zu einer Desaktivierung des Hydrierkatalysators führt (EP 394 967 A1, Seite 2 Zeile 34 ff, Seite 3 Zeile 44 ff). Es wird daher empfohlen, den Iminierungsschritt so durchzuführen, dass möglichst kein HCN abgespalten wird.

Gemäß EP 913 387 können zur Selektivitätssteigerung bei der Herstellung von IPDA auch quaternäre Ammoniumbasen eingesetzt werden. Entsprechend modifizierte Katalysatoren weisen speziell bei Verwendung eines Lösungsmittels eine deutlich höhere Standzeit auf als alkalimodifizierte Katalysatoren. WO 2005/039764 A offenbart einen Raney-Katalysator.

Weiterhin sind Verfahren zur Herstellung von Isophorondiamin aus CN 104230721A, EP 2649042A, WO 2008107226A und WO 2012126869A bekannt.

JP S62 123154 A offenbart offenbart ein Verfahren zur Herstellung von Isophoronnitril. JP H07 188126 A offenbart ein Verfahren zur Herstellung von Isophorondiamin aus Isophoronnitril.

Die zugrundeliegende Aufgabe war es, ein Verfahren zur Herstellung von 3-Aminomethyl-3,5,5-trimethylcyclohexylamin durch Hydrierung von Isophoronnitril zu finden, wobei die Ausbeute und Selektivität bei der reduktiven Aminierung von Isophoronnitril zur Herstellung von Isophorondiamin verbessert werden sollte.

Überraschend wurde ein neuer Katalysator wie unten näher beschrieben, gefunden. Als zusätzlicher unerwarteter Effekt konnten eine höhere Aktivität, wodurch niedrigere Reaktionstemperaturen möglich sind, und eine bessere Langzeitstabilität des Katalysators festgestellt werden.

Üblicherweise werden bei Verfahren zur Herstellung von 3-Aminomethyl-3,5,5-trimethylcyclohexylamin durch Hydrierung von Isophoronnitril Katalysatoren in Form von Pulvern oder Formkörpern, wie z. B. Extrudaten oder gepressten Pulvern, eingesetzt. Es können Vollkontakte, Raney-Typ-Katalysatoren oder Trägerkatalysatoren zur Anwendung kommen. Bevorzugt sind Raney-Typ- und Trägerkatalysatoren, wie in der EP 2649042A ausführlich beschrieben.

überraschend wurde nun gefunden, dass der erfindungsgemäß eingesetzte Katalysator bestehend aus einer Metall-Legierung in Granulautform bestimmter Partikelgrößen nach Aktivierung durch Laugen, die Augabe der Erfindung löst und zusätzlich eine höhere Aktivität und eine bessere Langzeitstabilität des Katalysators festgestellt wurden.

Gegenstand der Erfindung ist ein Verfahren zur Herstellung von Isophorondiamin dadurch gekennzeichnet, das
A) Isophoronnitril direkt in einer Stufe in Anwesenheit von Ammoniak, Wasserstoff, eines Hydrierkatalysator und gegebenenfalls weiteren Zusätzen und in An- oder Abwesenheit von organischen Lösungsmitteln aminierend zu Isophorondiamin hydriert wird;
   oder
B) Isophoronnitril in mindestens zwei oder mehreren Stufen zunächst ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

Und
II. Der Katalysator liegt in Form von unregelmäßigen Partikeln als Granulat vor und weist nach der Aktivierung Partikelgrößen von 1 bis 8 Millimeter (mm) gemessen durch Siebung. auf. Katalysator

Der Katalysator besteht aus einer Metall-Legierung, wobei die Metall-Legierung durch Basen auf der Oberfläche aktiviert ist. Die Schichtdicke der aktivierten Schicht auf der Partikeloberfläche des Katalysators beträgt bevorzugt 50 bis 1000 Mikrometer (µm). Sie kann aber auch größer oder kleiner sein. Auf der Oberfläche befindet sich demnach die katalytisch aktive Zusammensetzung des Katalysators. Es ist aber auch im Rahmen der Erfindung möglich, das gesamte Katalysator-Partikel fast vollständig oder vollständig auszulaugen.

Der erfindungsgemäß eingesetzte Katalysator liegt nach der Aktivierung als Granulat in Form einzelner Partikel vor.

Der erfindungsgemäß eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
1. Variante

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

2. Variante

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

Gesamtheit bedeutet, dass bei der Zusammensetzung nicht zwischen dem Gehalt der Metalle auf der Oberfläche und in der aktivierten Schicht und im Kern der Katalysator-Partikel unterschieden wird, sondern Alles zusammen addiert und berechnet wird.

Der Katalysator liegt in Form von unregelmäßigen Partikeln also als Granulat vor.

Zusätzlich weist der erfindungsgemäß eingesetzte Katalysator nach der Aktivierung die folgenden Partikelgrößen auf:
Im Allgemeinen weist der Katalysator, also die Granulat-Teilchen Partikelgrößen von 1 bis 8 Millimeter (mm) gemessen durch Siebung auf.

In einer ersten bevorzugten Variante der Erfindung variieren die Partikelgrößen des Katalysators, also die Granulat-Teilchen von 2,5 bis 6 Millimeter (mm).

In einer zweiten bevorzugten Variante der Erfindung variieren die Partikelgrößen des Katalysators, also die Granulat-Teilchen von 3 bis 7 Millimeter (mm).

In einer dritten bevorzugten Variante der Erfindung variieren die Partikelgrößen des Katalysators, also die Granulat-Teilchen von 2 bis 5 Millimeter (mm).

Die angegebenen Partikelgrößen können auch innerhalb der Bereiche eine statistische Verteilung der Größe aufweisen. Dabei sind enge Verteilungen als auch breite Verteilungen erfindungsgemäß.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition von Partikelgrößen, der Verteilung von Partikelgrößen und der Messung von Partikelgrößen in HORIBA^{®} Scientific, A GUIDEBOOK TO PARTICLE SIZE ANALYSIS, 2012, von HORIBAO Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden.

Bevorzugt wird der erfindungsgemäß eingesetzte Katalysator durch Siebung der hergestellten Granulate erhalten. Dabei werden sogenannte Siebfraktionen hergestellt. Dabei können einzelne Siebfraktionen gemischt werden, oder es wird ein Katalysator durch einmalige oder mehrmalige Siebung erhalten. Die so hergestellten Katalysatoren weisen bei den Partikelgrößen eine statistische Verteilung auf, üblicherweise in Form einer Gauß-Verteilung. Es sind symmetrische aber auch asymmetrische Verteilungen möglich.

In einer vierten bevorzugten Variante der Erfindung variieren die Partikelgrößen des Katalysators, also die Granulat-Teilchen mit einer statistischen Verteilung zwischen 2,5 bis 5,5 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

In einer fünften bevorzugten Variante der Erfindung variieren die Partikelgrößen des Katalysators, also die Granulat-Teilchen mit einer statistischen Verteilung zwischen 3,5 bis 6,5 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

In einer sechsten bevorzugten Variante der Erfindung variieren die Partikelgrößen des Katalysators, also die Granulat-Teilchen mit einer statistischen Verteilung zwischen 2 bis 5 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

In einer siebten bevorzugten Variante der Erfindung variieren die Partikelgrößen des Katalysators, also de Granulat-Teilchen mit einer statistischen Verteilung zwischen 3 bis 7 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

Geeignete Methoden und Beschreibungen zur Siebanalyse sind beschrieben in:
DIN 66165-1:1987-04 Partikelgrößenanalyse; Siebanalyse; Grundlagen, und in DIN 66165-2:1987-04 Partikelgrößenanalyse; Siebanalyse; Durchführung.
Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung.
Jörg Hoffmann: Handbuch der Messtechnik. Hanser Verlag, 2007, ISBN 978-3-446-40750-3, Kapitel 3.12.16.2.1.

Der erfindungsgemäß eingesetzte Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und mit
Partikelgrößen des Katalysators, also die Granulat-Teilchen mit einer statistischen Verteilung zwischen 2,5 bis 5,5 Millimeter (mm),
   oder
Partikelgrößen des Katalysators, also die Granulat-Teilchen mit einer statistischen Verteilung zwischen 3,5 bis 6,5 Millimeter (mm),
   oder
Partikelgrößen des Katalysators, also die Granulat-Teilchen mit einer statistischen Verteilung zwischen 2 bis 5 Millimeter (mm),
   oder
Partikelgrößen des Katalysators, also die Granulat-Teilchen mit einer statistischen Verteilung zwischen 3 bis 7 Millimeter (mm),
wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

Allgemeine Methode zur Herstellung des Katalysators:

### a) Herstellung der Legierung

Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.

### b) Herstellung der Granulate

Die Legierung wird in geeigneten Geräten zu Granulaten verarbeitet, zum Beispiel über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten (z. B. 3 - 7 mm).

### c) Aktivierung des Katalysators

Die Aktivierung des Katalysators erfolgt in geeigneten Apparaturen. Es können dabei organische oder anorganische Basen eingesetzt werden. Bevorzugt wird eine Lauge (z. B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 100 °C. Der Grad der Aktivierung wird über die Temperatur und die Reaktionszeit bestimmt. Dabei ist die Reaktionszeit variabel und ist abhängig von den Reaktionsbedingungen und dem gewünschten Grad der Aktivierung. Nach der Aktivierung wird der Katalysator mit Wasser gewaschen und anschließend unter Wasser gelagert.

Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Granulate erfolgen.

Zur Erhöhung der Aktivität, Selektivität und/oder Standzeit können die Katalysatoren zusätzlich Dotiermetalle oder andere Modifizierungsmittel enthalten. Typische Dotiermetalle sind z. B. Mo, Fe, Ag, V, Ga, In, Bi, Ti, Zr und Mn sowie die seltenen Erden, allein oder in Mischungen. Typische Modifizierungsmittel sind z. B. solche, mit denen die Säure-Base-Eigenschaften der Katalysatoren beeinflusst werden können, bevorzugt Alkali- und Erdalkalimetalle bzw. deren Verbindungen, bevorzugt Mg- und Li-Verbindungen. Für den Fall, dass derartige Verbindungen enthalten sind, in einer Menge von maximal ca. 5 Gew.-%, reduziert sich der Anteil der oben genannten Metall Co und AI sowie gegebenenfalls Cr und Ni im Katalysator entsprechend, wobei die Anteile an Co und AI sowie gegebenenfalls Cr und Ni dann nach der Aktivierung sich zu mindestens 95 Gew.-% addieren, bezogen auf die enthaltenden Metalle.

Es ist möglich, das erfindungsgemäße Verfahren in einer Stufe oder in mindestens zwei oder mehreren Stufen durchzuführen.

Wird das Verfahren in einer Stufe durchgeführt, wird Isophoronnitril direkt in Anwesenheit von Ammoniak, Wasserstoff, eines Katalysator und gegebenenfalls weiteren Zusätzen und in An- oder Abwesenheit von organischen Lösungsmitteln aminierend hydriert.

Der Begriff "in mindestens zwei oder in mehreren Stufen" bedeutet, dass zunächst in einem separaten Reaktor oder Reaktorabschnitt Isophoronnitril zunächst ganz oder teilweise in Isophoronnitrilimin umgewandelt wird, und dieses Isophoronnitrilimin als Reinsubstanz oder im Gemisch mit anderen Komponenten, wie zum Beispiel nicht umgesetztes Isophoronnitril, unter Anwesenheit von mindestens Ammoniak und Wasserstoff und eines Katalysators aminierend hydriert wird.

Eine bevorzugte Ausführungsform des erfindungsgemäßen Verfahrens zur Herstellung von IPDA ist ein zweistufiger Prozess: In der ersten Stufe wird zumindest ein Teil des eingesetzten IPN bei An- oder Abwesenheit eines Iminierungskatalysators und/oder von Lösungsmitteln durch Reaktion mit Ammoniak in Isophoronnitrilimin umgewandelt. Der Umsatz von IPN zu IPNI sollte nach der Iminierung größer 80 %, bevorzugt größer 90 %, besonders bevorzugt größer 95 % betragen.

In der zweiten Stufe wird das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert.

In einer weiteren bevorzugten Ausführungsform erfolgt die Umsetzung von IPN zu IPDA in drei voneinander getrennten Reaktionsräumen. In dem ersten Reaktionsraum erfolgt die Umsetzung von IPN zu Isophoronnitrilimin mit überschüssigem Ammoniak an Iminierungskatalysatoren bei Temperaturen zwischen 20 und 150 °C und Drücken zwischen 5 und 30 MPa. Im zweiten Reaktionsraum werden die entstandenen Reaktionsprodukte mit Wasserstoff in Gegenwart von überschüssigem Ammoniak an Hydrierkatalysatoren bei Temperaturen zwischen 20 und 130 °C und Drücken von 5 bis 30 MPa hydriert. Im dritten Reaktionsraum werden die entstanden Reaktionsprodukte an den erfindungsgemäß einzusetzenden Katalysatoren bei Temperaturen zwischen 100 und 160 °C und Drücken von 5 bis 30 MPa hydriert.

Um die Gleichgewichtseinstellung der Iminierungsreaktion zu beschleunigen ist es zweckmäßig, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionenaustauscher (siehe EP 042 119), geträgerte Heteropolysäuren (siehe DE 44 26 472), acide Metalloxide, insbesondere Aluminiumoxid und Titandioxid (siehe EP 449 089) Sulfonsäuregruppen enthaltende Organopolysiloxane (DE 196 27 265.3) und saure Zeolithe sowie Aktivkohle (EP 061 137). Bei Verwendung eines Iminierungskatalysators kann die Reaktionstemperatur zwischen 10 und 150 °C, vorzugsweise zwischen 30 und 130 °C und ganz besonders bevorzugt zwischen 40 und 100 °C liegen. Der Druck liegt zwischen dem Eigendruck der Mischung und 50 MPa. Bevorzugt wird die Iminierungsreaktion bei dem Druck durchgeführt, bei dem auch die anschließende reduktive Aminierung durchgeführt wird.

Obwohl die Iminierung von Isophoronnitril mit flüssigem Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol sowie Ether, besonders THF, MTBE und Dioxan.

In der Iminierungsstufe werden pro Mol eingesetztem IPN zwischen 1 und 500 Mol, bevorzugt 5 und 200 Mol, besonders bevorzugt zwischen 5 und 100 Mol Ammoniak eingesetzt. Typische Katalysatorbelastungen liegen im Bereich von 0,01 bis 10 kg IPN je kg Katalysator und Stunde, bevorzugt 0,5 bis 10 und besonders bevorzugt 0,5 bis 5 kg IPN je kg Katalysator und Stunde.

Bei der Iminierung in Gegenwart eines Iminierungskatalysators kann der Katalysator in Form eines Suspensionskatalysators oder Festbettkatalysators vorliegen. Vorteilhaft ist die Verwendung von Festbettkatalysatoren. In einer besonders bevorzugten Ausführungsform werden IPN und Ammoniak kontinuierlich von unten nach oben durch ein mit Iminierungskatalysator gefülltes Reaktionsrohr geleitet.

Die Hydrierung erfolgt in Festbettreaktoren. Geeignete Reaktortypen sind z. B. Schachtöfen, Hordenreaktoren oder Rohrbündelreaktoren.

Die Hydrierung erfolgt üblicherweise bei Temperaturen zwischen 20 und 150 °C, bevorzugt 40 und 130 °C, und Drücken von 0,3 bis 50 MPa, bevorzugt 5 bis 30 MPa. Es ist auch möglich, die Hydrierung in Gegenwart der bereits bei der Iminierungsstufe genannten Lösungsmittel durchzuführen. Der wesentliche Vorteil bei Verwendung eines Lösungsmittels liegt darin, dass die Hydrierung bei niedrigeren Drücken zwischen 0,3 und 10 MPa durchgeführt werden kann.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung in flüssigem Ammoniak als Lösungsmittel. Pro Mol IPN werden zwischen 1 und 500 Mol, bevorzugt 5 und 200 Mol, besonders bevorzugt zwischen 5 und 100 Mol Ammoniak verwendet. Zweckmäßigerweise verwendet man mindestens die Ammoniakmenge, die bei der vorgelagerten Iminierung eingestellt wurde. Der Ammoniakanteil kann aber auch vor der Hydrierung durch Zugabe von zusätzlichem Ammoniak auf den gewünschten Wert erhöht werden.

Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN, Ammoniak und Wasserstoff zwischen 0,5 und 5 Liter IPN/Ammoniak-Mischung pro Liter Katalysator und Stunde, bevorzugt zwischen 1 und 4 I_{Lsg} I_{Kat}⁻¹ h⁻¹.

Es ist bevorzugt, dass die einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 m³ Ammoniak pro m³ Katalysator und Stunde eingesetzt. Üblicherweise wird bei Temperaturen zwischen 20 und 150 °C, bevorzugt 40 bis 130 °C, gearbeitet. Besonders bevorzugt wird eine Temperaturrampe durchfahren, bei der der Katalysator beginnend bei mäßig erhöhter Temperatur, bevorzugt zwischen 20 und 50 °C, langsam bis auf die später für die Hydrierung gewünschte Reaktionstemperatur, bevorzugt 20 bis 150 °C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 50 MPa, bevorzugt 5 bis 40 MPa, besonders bevorzugt 10 bis 30 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

In dem bevorzugten zweistufigen Prozess wird das Isophoronnitrilimin enthaltende Gemisch in Gegenwart des Hydrierkatalysators in der zweiten Stufe hydriert. Das der Hydrierstufe zugeführte Gemisch kann unmittelbar jenes sein, welches bei der Iminierung von IPN mit Ammoniak in der der ersten Stufe anfällt, oder wie es nach Zugabe oder Entfernung von Komponenten, wie z. B. Ammoniak, organischen Lösungsmitteln, Basen, Cyanidsalze, Blausäure und/oder Wasser, erhalten wird. Bevorzugt wird die Hydrierung kontinuierlich in Festbettreaktoren durchgeführt, die in Riesel- oder Sumpffahrweise betrieben werden können. Geeignete Reaktortypen sind z. B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Außer den zuvor genannten Bestandteilen des der Iminierungsstufe zuzuführenden Gemisches kann dieses zusätzlich höher oder niedriger als IPDA siedende Fraktionen aus der destillativen Aufarbeitung des aus dem Rieselbettreaktor abgezogenen Reaktionsgemisches enthalten. Derartige Fraktionen können außer Resten an IPDA auch solche Nebenprodukte enthalten, aus denen sich unter Reaktionsbedingungen erneut IPDA bildet. Besonders vorteilhaft ist es, die oberhalb IPDA siedende Fraktion, welche außer Resten an IPDA 2-Aza-4,6,6-trimethylbicyclo[3.2.1]-octan als Hauptprodukt enthält, zurückzuführen. Ebenfalls besonders vorteilhaft ist es, nicht vollständig umgesetztes IPN, insbesondere Isophoronaminonitril enthaltende Fraktionen, zurückzuführen. Die Rückführungen können auch, falls dieses gewünscht ist, unmittelbar dem der Hydrierstufe zuzuführenden Reaktionsgemisch zugesetzt werden.

Bei der Hydrierung von IPN bzw. Isophoronnitrilimin können zwei unterschiedliche Stereoisomere gebildet werden. Durch die Wahl eines Temperaturprofils im Hydrierschritt lässt sich das Isomerenverhältnis beeinflussen. Es ist z. B. möglich, ein IPN oder Isophoronnitrilimin enthaltendes Gemisch zunächst bei einer Temperatur zwischen 20 und 90 °C teilweise zu hydrieren, und anschließend die Reaktion in einem zweiten Schritt in einem Temperaturbereich zwischen 90 und 150 °C zu vervollständigen. Durch die Einhaltung relativ niedriger Reaktionstemperaturen im 1. Schritt kann die Selektivität zu Gunsten des cis-Isomeren verschoben werden. Die Einhaltung relativ niedriger Reaktionstemperaturen zu Beginn der Reaktion hat zudem den Vorteil, dass das thermisch labile Isophoronnitrilimin besonders schonend hydriert wird und dadurch Nebenreaktionen unterbunden werden. Das intermediär gebildete Isophoronaminonitril ist thermisch deutlich stabiler und kann daher bei höheren Temperaturen hydriert werden, ohne dass weitere Nebenreaktionen befürchtet werden müssen. Zu den unerwünschten Nebenreaktionen zählt auch die Abspaltung von HCN. Nach dem erfindungsgemäßen Verfahren wirkt sich eine gewisse Cyanidionenkonzentration positiv auf die Selektivität der Hydrierstufe aus. Dieser Effekt tritt verstärkt dann zu Tage, wenn die Cyanidionen schon von Anfang an in der Hydrierstufe vorliegen und nicht erst während der Hydrierung entstehen. Daher ist eine Abspaltung von HCN während der Hydrierstufe zu verhindern.

Die Realisierung des gewünschten Temperaturprofils kann beispielsweise durch die Hintereinanderschaltung von zwei oder mehr getrennt voneinander beheizbaren Reaktoren erfolgen. Es ist aber auch möglich, ein ansteigendes Temperaturprofil in nur einem Hydrierreaktor zu realisieren. Besonders bevorzugt erfolgt die Durchführung der Hydrierreaktion in einem adiabatisch betriebenen Rieselbettreaktor, bei dem das Reaktionsgemisch dem Reaktor bei Temperaturen zwischen 20 und 90 °C zugeführt wird, und aufgrund der auftretenden und durch das Reaktionsgemisch aufgenommenen Reaktionswärme zwischen 90 und 150 °C wieder verlässt.

Das die Hydrierung verlassende Reaktionsgemisch wird mit den üblichen Methoden weiter aufgereinigt, um ein IPDA mit der gewünschten Qualität zu erhalten. Dabei können alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden. Mögliche Komponenten, die beispielsweise bei der weiteren Aufreinigung abgetrennt werden, sind Wasserstoff, Ammoniak, Wasser sowie die bei der Herstellung von IPDA aus IPN anfallenden Nebenprodukte wie z. B. hydrierte HCN-Eliminierungsprodukte oder Verunreinigungen des IPN, methylierte Nebenprodukte und/oder unvollständig hydrierte Zwischenprodukte.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z. B. Dephlegmatoren, Trennwänden, ungeordnete Einbauten oder Schüttungen, geordnete Einbauten oder Packungen, Böden mit oder ohne Zwangsführung verwenden.

In einem ersten Schritt werden insbesondere Wasserstoff, Inertgase, Ammoniak, leicht siedende Verunreinigungen und ggf. auch Wasser in einer oder mehreren Destillationskolonnen ganz oder teilweise abgetrennt. Dabei erfolgt die Abtrennung bevorzugt bei einem Druck, der kleiner ist als im Reaktionsschritt. Erfolgt die Abtrennung in mehreren Destillationsschritten, so ist es vorteilhaft, das der Druck stufenweise abgesenkt wird. Ganz besonders bevorzugt erfolgt die Abtrennung oberhalb 1 bar und mit Sumpftemperaturen von 0 - 200 °C. Der Einsatz eines Stripgases zur Entfernung von leicht siedenden Verunreinigungen kann vorteilhaft sein. Insbesondere Ammoniak und Wasserstoff und Anteile der leicht siedenden Verunreinigungen können ganz oder zum Teil in den Prozess (Reaktion) zurückgeführt werden. Die leicht siedenden Verunreinigungen und gegebenenfalls Anteile von Wasserstoff und Ammoniak werden der thermischen Verwertung zugeführt.

In einem zweiten Schritt werden weitere leicht siedende Verunreinigungen, Wasser und schwer siedende Verunreinigungen ganz oder teilweise abgetrennt. Dies kann in einer oder mehreren Destillationskolonnen erfolgen. Dabei kann Wasser zusammen mit organischen, leicht siedenden Verunreinigungen und gegebenenfalls Anteilen an IPDA über Kopf der Kolonne abdestilliert werden und nach Kondensation in eine wässrige und eine organische Phase getrennt werden. Hierbei kann die organische Phase teilweise als Rückfluss in die Kolonne zurückgeführt werden. Wird der zweite Schritt der Destillation in einer einzigen Kolonne durchgeführt (z. B. einer Trennwandkolonne), so wird das IPDA mit der gewünschten Reinheit über einen Seitenstrom entnommen, während die schwer siedenden Verunreinigungen im Sumpf der Kolonne anfallen. Wird die Trennung aber zwei- oder mehrstufig durchgeführt, so wird das IPDA am Kopf einer Kolonne erhalten. Die Abtrennung der leicht und schwer siedenden Verunreinigungen und von Wasser erfolgt bevorzugt im Vakuum zwischen 100 Pa und 0,0999 MPa und Sumpftemperaturen von 50 - 300 °C. Sämtliche Nebenkomponenten können der thermischen Verwertung zugeführt werden.

### Beispiel

### Herstellung des Katalysators, Co-Granulat:

### c) Herstellung der Legierung

Die Herstellung der Legierung erfolgt in einem Induktionsofen. Es werden dabei die Metalle in den entsprechenden Mengen bei 1500°C geschmolzen. Die fertige Schmelze wird für die Weiterverarbeitung zu Barren gegossen.

### d) Herstellung der Granulate

Die Legierungsbarren werden über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten.

### a) Aktivierung des Katalysators

Die Aktivierung des Katalysators erfolgt in einer Standard-Glaslaborapparatur, z.B. einem Becherglas. Zu den Granulaten wurde unter Rühren eine wässrige Lauge (z.B. Natronlauge) gegeben. Die Granulate befinden sich während der Aktivierung in einem Katalysatorkorb. Durch einen exothermen Vorgang wird ein Teil des Aluminiums unter Bildung von Wasserstoff und Natriumaluminatlauge aus der Legierung herausgelöst. Die Konzentration der eingesetzten Lauge lag bei 20 gew.-% und die Reaktionstemperatur bei 90 °C. Der Grad der Aktivierung wurde über die Reaktionszeit bestimmt. Nach der Aktivierung wird der Katalysator mit Wasser gewaschen und anschließend unter Wasser gelagert.

Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 75,9 Gew.-% |
| Aluminium: | 20,0 Gew.-% |
| Chrom: | 1,5 Gew.-% |
| Nickel: | 2,6 Gew.-% |

Es wurde eine Siebfraktion eingesetzt mit Partikelgrößen des Katalysators, also die Granulat-Teilchen mit einer statistischen Verteilung zwischen 2,0 bis 5,0 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

### Herstellung von IPDA mit dem erfindungsgemäß eingesetzten Katalysator

Die Katalysatoren werden bei der Herstellung von 3-Aminomethyl-3,5,5-Trimethylcyclohexylamin (Isophorondiamin, IPDA) aus 3-Cyano-3,5,5-Trimethylcyclohexanon (Isophoronnitril, IPN) in einem zweistufigen Verfahren auf ihr katalytische Wirksamkeit getestet.

In der ersten Stufe wird dabei Isophoronnitril in Anwesenheit eines Iminierungskatalysators mit Ammoniak zumindest teilweise zu 3-Cyano-3,5,5-Trimethylcyclohexanimin überführt und in der zweiten Stufe an einem Hydrierungskatalysator bei einer Temperatur von 60-100°C und einem Druck von 250 bar mit Wasserstoff unter Anwesenheit von Ammoniak aminierend hydriert. Jede Stufe der Herstellung wird in einem separaten Reaktor durchgeführt. Beide Reaktoren sind jedoch hintereinander geschaltet und werden einzeln temperiert.

Der Hydrierreaktor wird mit 42 ml des zu prüfenden Katalysators befüllt. Die Einsatzlösung aus IPN (15 Gew.-%) und Ammoniak (85 Gew.-%) wird mit einem Massenstrom von 110 ml/h von oben nach unten durch das Reaktionsrohr gepumpt. Der Wasserstoff wird separat mit einem Volumenstrom von 40 l/h ebenfalls von oben zugegeben. Die Produktflüssigkeit wird unter dem Reaktor in einem Abscheidegefäß aufgefangen. Die gesammelte Produktmischung wird auf IPDA und entsprechende Nebenkomponenten gaschromatographisch untersucht. Die Ergebnisse sind in Tabelle 1 aufgeführt.

**Tabelle 1**

| Temperatur | Ausbeute IPDA / GC-% | Umsatz / % |
|---|---|---|
| 100 °C | 98,1 | 99,5 |
| 80 °C | 96,7 | 98,5 |
| 60 °C | 92,1 | 93,1 |

### Langzeitstabilität

In der ersten Stufe wird Isophoronnitril in Anwesenheit eines Iminierungskatalysators mit Ammoniak zumindest teilweise zu 3-Cyano-3,5,5-Trimethylcyclohexanimin überführt und in der zweiten Stufe an einem Hydrierungskatalysator bei einer Temperatur von 60 °C (erfindungsgemäßer Katalysator) bzw. 100°C (Referenzkatalysator, geträgerter gepresster Co-Formkörper) und einem Druck von 250 bar mit Wasserstoff unter Anwesenheit von Ammoniak aminierend hydriert. Jede Stufe der Herstellung wird in einem separaten Reaktor durchgeführt. Beide Reaktoren sind jedoch hintereinander geschaltet und werden einzeln temperiert.

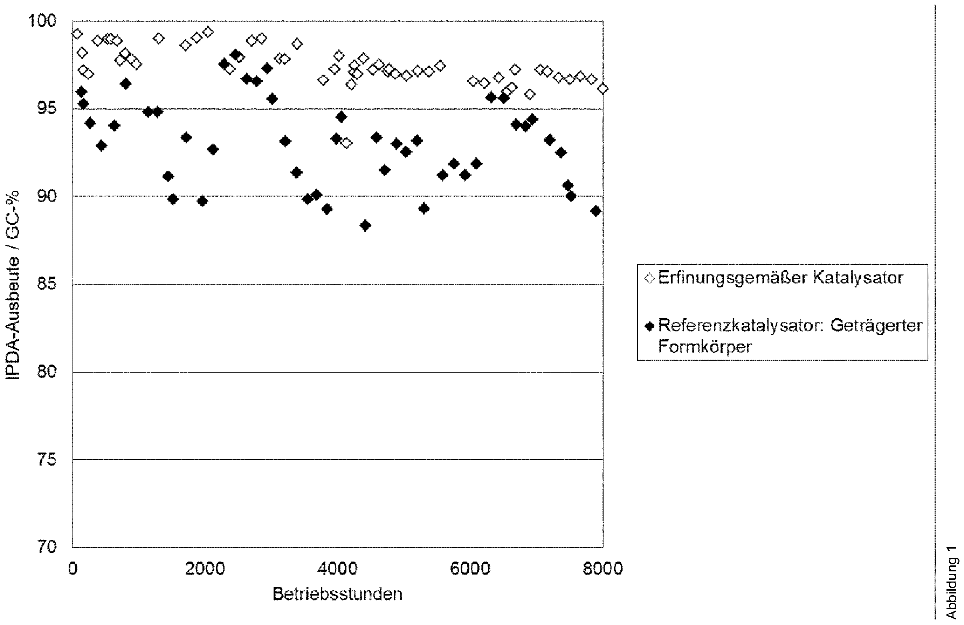

Für die Überprüfung der Langzeitstabilität wird der Hydrierreaktor mit 100 ml des zu prüfenden Katalysator befüllt. Die Einsatzlösung aus IPN (22 Gew.-%) und Ammoniak (78 Gew.-%) wird mit einem Massenstrom von 120 g/h von oben nach unten durch das Reaktionsrohr gepumpt. Zusätzlich wird Wasserstoff mit einem Volumenstrom von 50 l/h ebenfalls von oben zugegeben. Die Produktflüssigkeit wird unter dem Reaktor in einem Abscheidegefäß aufgefangen. Die gesammelte Produktmischung wird auf IPDA und entsprechende Nebenkomponenten gaschromatographisch untersucht. Die Ergebnisse sind in Abbildung 1 aufgeführt.

Erfindungsgemäß eingesetzter Katalysator Co-Granulat der Zusammensetzung wie oben beschrieben Referenzkatalysator: Geträgerter gepresster Co-Katalysator-Forrmkörper

## Patentansprüche

1. Verfahren zur Herstellung von Isophorondiamin, **dadurch gekennzeichnet**,
A) Isophoronnitril direkt in einer Stufe in Anwesenheit von Ammoniak, Wasserstoff, eines Hydrierkatalysator und gegebenenfalls weiteren Zusätzen und in An- oder Abwesenheit von organischen Lösungsmitteln aminierend zu Isophorondiamin hydriert wird;
oder
B) Isophoronnitril in mindestens zwei oder mehreren Stufen zunächst ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |
und
II. Der Katalysator liegt in Form von unregelmäßigen Partikeln als Granulat vor und weist nach der Aktivierung Partikelgrößen von 1 bis 8 Millimeter (mm) gemessen durch Siebung auf.

2. Verfahren zur Herstellung von Isophorondiamin nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

3. Verfahren zur Herstellung von Isophorondiamin nach mindestens einem der vorherigen Ansprüche 1-2, **dadurch gekennzeichnet, dass**
die Partikelgrößen des Katalysators, also die Granulat-Teilchen von 2,5 bis 6 Millimeter (mm) variieren,
oder
die Partikelgrößen des Katalysators, also die Granulat-Teilchen von 3 bis 7 Millimeter (mm) variieren,
oder
die Partikelgrößen des Katalysators, also die Granulat-Teilchen von 2 bis 5 Millimeter (mm) variieren.

4. Verfahren zur Herstellung von Isophorondiamin nach mindestens einem der vorherigen Ansprüche 1-2, **dadurch gekennzeichnet, dass** der Katalysator durch Siebung der hergestellten Granulate erhalten wird.

5. Verfahren zur Herstellung von Isophorondiamin nach Anspruch 4, **dadurch gekennzeichnet, dass** der Katalysator durch Siebung der hergestellten Granulate erhalten wird, und
die Partikelgrößen des Katalysators, also die Granulat-Teilchen mit einer statistischen Verteilung zwischen 2,5 bis 5,5 Millimeter (mm), liegen, oder
die Partikelgrößen des Katalysators, also die Granulat-Teilchen mit einer statistischen Verteilung zwischen 3,5 bis 6,5 Millimeter (mm), liegen, oder
die Partikelgrößen des Katalysators, also die Granulat-Teilchen mit einer statistischen Verteilung zwischen 2 bis 5 Millimeter (mm), liegen, oder
die Partikelgrößen des Katalysators, also de Granulat-Teilchen mit einer statistischen Verteilung zwischen 3 bis 7 Millimeter (mm),
und wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

6. Verfahren zur Herstellung von Isophorondiamin nach Anspruch 1, **dadurch gekennzeichnet, dass** der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |
und mit
Partikelgrößen des Katalysators, also die Granulat-Teilchen mit einer statistischen Verteilung zwischen 2,5 bis 5,5 Millimeter (mm),
oder
Partikelgrößen des Katalysators, also die Granulat-Teilchen mit einer statistischen Verteilung zwischen 3,5 bis 6,5 Millimeter (mm),
oder
Partikelgrößen des Katalysators, also die Granulat-Teilchen mit einer statistischen Verteilung zwischen 2 bis 5 Millimeter (mm),
oder
Partikelgrößen des Katalysators, also die Granulat-Teilchen mit einer statistischen Verteilung zwischen 3 bis 7 Millimeter (mm),
wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

7. Verfahren zur Herstellung von Isophorondiamin nach mindestens einem der vorherigen Ansprüche, **dadurch gekennzeichnet, dass** die Katalysatoren zusätzlich Dotiermetalle enthalten, bevorzugt ausgewählt aus Mo, Fe, Ag, V, Ga, In, Bi, Ti, Zr und Mn, sowie den seltenen Erden.

8. Verfahren zur Herstellung von Isophorondiamin nach mindestens einem der vorherigen Ansprüche, **dadurch gekennzeichnet, dass** Katalysatoren Modifizierungsmittel enthalten, bevorzugt Alkali- und Erdalkalimetalle bzw. deren Verbindungen, bevorzugt Mg- und Li-Verbindungen.

9. Verfahren zur Herstellung von Isophorondiamin nach mindestens einem der vorherigen Ansprüche, **dadurch gekennzeichnet, dass** in der ersten Stufe wird zumindest ein Teil des eingesetzten Isophoronnitrilimin bei An- oder Abwesenheit eines Iminierungskatalysators und/oder von Lösungsmitteln durch Reaktion mit Ammoniak in Isophoronnitrilimin umgewandelt, wobei der Umsatz von IPN zu IPNI nach der Iminierung größer 80 %, bevorzugt größer 90 %, besonders bevorzugt größer 95 % beträgt.

10. Verfahren zur Herstellung von Isophorondiamin nach mindestens einem der vorherigen Ansprüche, **dadurch gekennzeichnet, dass** in der zweiten Stufe das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert wird.

11. Verfahren zur Herstellung von Isophorondiamin nach mindestens einem der vorherigen Ansprüche, **dadurch gekennzeichnet, dass** die Umsetzung von IPN zu IPDA in drei voneinander getrennten Reaktionsräumen erfolgt, wobei in dem ersten Reaktionsraum die Umsetzung von IPN zu Isophoronnitrilimin mit überschüssigem Ammoniak an Iminierungskatalysatoren bei Temperaturen zwischen 20 und 150 °C und Drücken zwischen 5 und 30 MPa erfolgt, im zweiten Reaktionsraum die entstandenen Reaktionsprodukte mit Wasserstoff in Gegenwart von überschüssigem Ammoniak an Hydrierkatalysatoren bei Temperaturen zwischen 20 und 130 °C und Drücken von 5 bis 30 MPa hydriert werden, und im dritten Reaktionsraum die entstanden Reaktionsprodukte an Katalysatoren bei Temperaturen zwischen 100 und 160 °C und Drücken von 5 bis 30 MPa hydriert werden.

12. Verfahren zur Herstellung von Isophorondiamin nach mindestens einem der vorherigen Ansprüche, **dadurch gekennzeichnet, dass** die Iminierungsreaktion in Gegenwart mindestens eines Iminierungskatalysators erfolgt.

13. Verfahren zur Herstellung von Isophorondiamin nach mindestens einem der vorherigen Ansprüche, **dadurch gekennzeichnet, dass** die Iminierung von Isophoronnitril mit flüssigem Ammoniak ohne Zugabe weiterer Lösungsmittel durchgeführt wird.

14. Verfahren zur Herstellung von Isophorondiamin nach mindestens einem der vorherigen Ansprüche, **dadurch gekennzeichnet, dass** in der Iminierungsstufe pro Mol eingesetztem IPN zwischen 1 und 500 Mol, bevorzugt 5 und 200 Mol, besonders bevorzugt zwischen 5 und 100 Mol Ammoniak eingesetzt werden.

15. Verfahren zur Herstellung von Isophorondiamin nach mindestens einem der vorherigen Ansprüche, **dadurch gekennzeichnet, dass**
die Iminierung in Gegenwart eines Suspensionskatalysators oder Festbettkatalysators, bevorzugt mindestens eines Festbettkatalysators, durchgeführt wird.

16. Verfahren zur Herstellung von Isophorondiamin nach mindestens einem der vorherigen Ansprüche, **dadurch gekennzeichnet, dass** bei der Iminierung IPN und Ammoniak kontinuierlich von unten nach oben durch ein mit Iminierungskatalysator gefülltes Reaktionsrohr geleitet werden.

17. Verfahren zur Herstellung von Isophorondiamin nach mindestens einem der vorherigen Ansprüche, **dadurch gekennzeichnet, dass** der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

18. Verfahren zur Herstellung von Isophorondiamin nach mindestens einem der vorherigen Ansprüche, **dadurch gekennzeichnet, dass** die Hydrierung in flüssigem Ammoniak als Lösungsmittel durchgeführt wird, wobei. pro Mol IPN werden zwischen 1 und 500 Mol, bevorzugt 5 und 200 Mol, besonders bevorzugt zwischen 5 und 100 Mol Ammoniak eingesetzt werden.

19. Verfahren zur Herstellung von Isophorondiamin nach mindestens einem der vorherigen Ansprüche, **dadurch gekennzeichnet, dass** die Hydrierkatalysatoren vor ihrem Einsatz in der Hydrierung zunächst mit Ammoniak konditioniert werden.

20. Verfahren zur Herstellung von Isophorondiamin nach mindestens einem der vorherigen Ansprüche, **dadurch gekennzeichnet, dass** Hydrierung kontinuierlich in Festbettreaktoren erfolgt.

21. Verfahren zur Herstellung von Isophorondiamin nach mindestens einem der vorherigen Ansprüche, **dadurch gekennzeichnet, dass** die Hydrierung kontinuierlich in Festbettreaktoren, die in Rieselbettfahrweise oder Sumpffahrweise betrieben werden, durchgeführt wird.

22. Verfahren zur Herstellung von Isophorondiamin nach mindestens einem der vorherigen Ansprüche, **dadurch gekennzeichnet, dass** das die Hydrierung verlassende Reaktionsgemisch einstufig oder mehrstufig aufgereinigt wird, und das Isophorondiamin erhalten wird.

23. Verfahren zur Herstellung von Isophorondiamin nach mindestens einem der vorherigen Ansprüche, **dadurch gekennzeichnet, dass** das die Hydrierung verlassende Reaktionsgemisch in zwei Schritten aufgereinigt wird, wobei in einem ersten Schritt insbesondere Wasserstoff, Inertgase, Ammoniak, leicht siedende Verunreinigungen und gegebenenfalls Wasser in einer oder mehreren Destillationskolonnen ganz oder teilweise abgetrennt wird und in einem zweiten Schritt weitere leicht siedende Verunreinigungen, Wasser und schwer siedende Verunreinigungen ganz oder teilweise in Destillationskolonnen abgetrennt werden, und das Isophorondiamin erhalten wird.

24. Verwendung eines Katalysators zur Herstellung von Isophorondiamin, **dadurch gekennzeichnet, dass**
A) Isophoronnitril direkt in einer Stufe in Anwesenheit von Ammoniak, Wasserstoff, eines Hydrierkatalysator und gegebenenfalls weiteren Zusätzen und in An- oder Abwesenheit von organischen Lösungsmitteln aminierend zu Isophorondiamin hydriert wird;
oder
B) Isophoronnitril in mindestens zwei oder mehreren Stufen zunächst ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |
und
II. Der Katalysator liegt in Form von unregelmäßigen Partikeln als Granulat vor und weist nach der Aktivierung Partikelgrößen von 1 bis 8 Millimeter (mm) gemessen durch Siebung auf.

## Claims

1. Process for preparing isophoronediamine, **characterized in that**
A) isophoronenitrile is subjected directly in one stage to aminating hydrogenation to give isophoronediamine in the presence of ammonia, hydrogen, a hydrogenation catalyst and possibly further additions, and in the presence or absence of organic solvents;
or
B) isophoronenitrile is first converted fully or partly in at least two or more than two stages to isophoronenitrile imine, and this isophoronenitrile imine is subjected to aminating hydrogenation to give isophoronediamine as a pure substance or in a mixture with other components and/or isophoronenitrile, in the presence of at least ammonia, hydrogen and a catalyst, wherein the catalyst has the following properties:
I. The catalyst has, after the activation, in its entirety, the following composition in per cent by weight (% by weight), where the proportions add up to 100% by weight, based on the metals present:
Cobalt: 55% to 85% by weight
Aluminium: 5% to 43.5% by weight
Chromium: 0.5% to 3% by weight
Nickel: 1% to 7% by weight
and
II. The catalyst is in the form of irregular particles as a granular material and after activation has particle sizes of 1 to 8 millimetres (mm) measured by screening.

2. Process for preparing isophoronediamine according to Claim 1, **characterized in that**
I. The catalyst has, after the activation, in its entirety, the following composition in per cent by weight (% by weight), where the proportions add up to 100% by weight, based on the metals present:
Cobalt: 57% to 84% by weight
Aluminium: 10% to 40% by weight
Chromium: 1% to 2% by weight
Nickel: 2% to 4% by weight

3. Process for preparing isophoronediamine according to at least one of the preceding Claims 1-2, **characterized in that**
the particle sizes of the catalyst, i.e. the granulate particles, vary from 2.5 to 6 millimetres (mm),
or
the particle sizes of the catalyst, i.e. the granulate particles, vary from 3 to 7 millimetres (mm),
or
the particle sizes of the catalyst, i.e. the granulate particles, vary from 2 to 5 millimetres (mm).

4. Process for preparing isophoronediamine according to at least one of the preceding Claims 1-2, **characterized in that** the catalyst is obtained by screening the granules produced.

5. Process for preparing isophoronediamine according to Claim 4, **characterized in that** the catalyst is obtained by screening the granules produced, and
the particle sizes of the catalyst, i.e. the granulate particles, having a statistical distribution between 2.5 to 5.5 millimetres (mm), or
the particle sizes of the catalyst, i.e. the granulate particles, having a statistical distribution between 3.5 to 6.5 millimetres (mm), or
the particle sizes of the catalyst, i.e. the granulate particles, having a statistical distribution between 2 to 5 millimetres (mm), or
the particle sizes of the catalyst, i.e. the granulate particles, having a statistical distribution between 3 to 7 millimetres (mm),
and where up to 10 per cent of the particles may also be outside said range of said lower limit or upper limit, but up to 10 percent in each case may also be outside said range of said lower limit and upper limit.

6. Process for preparing isophoronediamine according to Claim 1, **characterized in that** the catalyst has, after the activation, in its entirety, the following composition in per cent by weight (% by weight), where the proportions add up to 100% by weight, based on the metals present:
Cobalt: 57% to 84% by weight
Aluminium: 10% to 40% by weight
Chromium: 1% to 2% by weight
Nickel: 2% to 4% by weight
and comprising
particle sizes of the catalyst, i.e. the granulate particles, having a statistical distribution between 2.5 to 5.5 millimetres (mm),
or
particle sizes of the catalyst, i.e. the granulate particles, having a statistical distribution between 3.5 to 6.5 millimetres (mm),
or
particle sizes of the catalyst, i.e. the granulate particles, having a statistical distribution between 2 to 5 millimetres (mm),
or
particle sizes of the catalyst, i.e. the granulate particles, having a statistical distribution between 3 to 7 millimetres (mm),
where up to 10 per cent of the particles may also be outside said range of said lower limit or upper limit, but up to 10 per cent in each case may also be outside said range of said lower limit and upper limit.

7. Process for preparing isophoronediamine according to at least one of the preceding claims, **characterized in that** the catalysts additionally comprise doping metals, preferably selected from Mo, Fe, Ag, V, Ga, In, Bi, Ti, Zr and Mn, and the rare earths.

8. Process for preparing isophoronediamine according to at least one of the preceding claims, **characterized in that** catalysts comprise modifiers, preferably alkali metals and alkaline earth metals or compounds thereof, preferably magnesium and lithium compounds.

9. Process for preparing isophoronediamine according to at least one of the preceding claims, **characterized in that** at least some of the isophoronenitrile imine used is converted to isophoronenitrile imine in the first stage by reaction with ammonia in the presence or absence of an imination catalyst and/or of solvents, the conversion of IPN to IPNI after the imination being greater than 80%, preferably greater than 90%, particularly preferably greater than 95%.

10. Process for preparing isophoronediamine according to at least one of the preceding claims, **characterized in that** the first stage reaction product, as obtained or after a further treatment and/or addition of further ammonia, is subjected in the second stage to aminating hydrogenation over hydrogenation catalysts in the presence of at least ammonia and hydrogen and in the presence or absence of an organic solvent at a temperature of 20 to 150°C, preferably 40 to 130°C, and a pressure of 0.3 to 50 MPa, preferably 5 to 30 MPa.

11. Process for preparing isophoronediamine according to at least one of the preceding claims, **characterized in that** the conversion of IPN to IPDA is effected in three separate reaction spaces, IPN being converted to isophoronenitrile imine with excess ammonia over imination catalysts in the first reaction space at temperatures between 20 and 150°C and pressures between 5 and 30 MPa, the reaction products formed being hydrogenated with hydrogen in the presence of excess ammonia over hydrogenation catalysts in the second reaction space at temperatures between 20 and 130°C and pressures of 5 to 30 MPa, and the reaction products formed being hydrogenated over catalysts in the third reaction space at temperatures between 100 and 160°C and pressures of 5 to 30 MPa.

12. Process for preparing isophoronediamine according to at least one of the preceding claims, **characterized in that** the imination reaction is effected in the presence of at least one imination catalyst.

13. Process for preparing isophoronediamine according to at least one of the preceding claims, **characterized in that** the imination of isophoronenitrile with liquid ammonia is conducted without addition of further solvent.

14. Process for preparing isophoronediamine according to at least one of the preceding claims, **characterized in that** between 1 and 500 mol, preferably 5 and 200 mol, more preferably between 5 and 100 mol, of ammonia is used per mole of IPN used in the imination stage.

15. Process for preparing isophoronediamine according to at least one of the preceding claims, **characterized in that**
the imination is conducted in the presence of a suspension catalyst or fixed bed catalyst, preferably of at least one fixed bed catalyst.

16. Process for preparing isophoronediamine according to at least one of the preceding claims, **characterized in that** IPN and ammonia in the imination are conducted continuously from the bottom upward through a reaction tube filled with imination catalyst.

17. Process for preparing isophoronediamine according to at least one of the preceding claims, **characterized in that** the hydrogen required for the hydrogenation is supplied to the reactor either in excess, preferably at up to 10 000 molar equivalents, or in such an amount that the hydrogen consumed by reaction and the portion of the hydrogen which leaves the reactor dissolved in the product stream is replenished.

18. Process for preparing isophoronediamine according to at least one of the preceding claims, **characterized in that** the hydrogenation is conducted in liquid ammonia as solvent, using between 1 and 500 mol, preferably 5 and 200 mol, more preferably between 5 and 100 mol, of ammonia per mole of IPN.

19. Process for preparing isophoronediamine according to at least one of the preceding claims, **characterized in that** the hydrogenation catalysts are first conditioned with ammonia before they are used in the hydrogenation.

20. Process for preparing isophoronediamine according to at least one of the preceding claims, **characterized in that** hydrogenation is effected continuously in fixed bed reactors.

21. Process for preparing isophoronediamine according to at least one of the preceding claims, **characterized in that** the hydrogenation is conducted continuously in fixed bed reactors which are operated in trickle mode or liquid phase mode.

22. Process for preparing isophoronediamine according to at least one of the preceding claims, **characterized in that** the reaction mixture leaving the hydrogenation is purified in one or more stages, and the isophoronediamine is obtained.

23. Process for preparing isophoronediamine according to at least one of the preceding claims, **characterized in that** the reaction mixture leaving the hydrogenation is purified in two steps, with complete or partial removal particularly of hydrogen, inert gases, ammonia, low-boiling impurities and optionally water in one or more distillation columns in a first step, and complete or partial removal of further low-boiling impurities, water and high-boiling impurities in distillation columns in a second step, and the isophoronediamine is obtained.

24. Use of a catalyst for preparation of isophoronediamine, **characterized in that**
A) isophoronenitrile is subjected directly in one stage to aminating hydrogenation to give isophoronediamine in the presence of ammonia, hydrogen, a hydrogenation catalyst and possibly further additions, and in the presence or absence of organic solvents;
or
B) isophoronenitrile is first converted fully or partly in at least two or more than two stages to isophoronenitrile imine, and this isophoronenitrile imine is subjected to aminating hydrogenation to give isophoronediamine as a pure substance or in a mixture with other components and/or isophoronenitrile, in the presence of at least ammonia, hydrogen and a catalyst, wherein the catalyst has the following properties:
I. The catalyst has, after the activation, in its entirety, the following composition in per cent by weight (% by weight), where the proportions add up to 100% by weight, based on the metals present:
Cobalt: 55% to 85% by weight
Aluminium: 5% to 43.5% by weight
Chromium: 0.5% to 3% by weight
Nickel: 1% to 7% by weight
and
II. The catalyst is in the form of irregular particles as a granular material and after activation has particle sizes of 1 to 8 millimetres (mm) measured by screening.

## Revendications

1. Procédé de préparation d'isophoronediamine, **caractérisé en ce que**
A) de l'isophoronenitrile est hydrogéné avec amination, directement en une étape en présence d'ammoniac, d'hydrogène, d'un catalyseur d'hydrogénation et le cas échéant d'autres additifs et en présence ou en l'absence de solvants organiques en isophoronediamine ; ou
B) de l'isophoronenitrile est converti en au moins deux étapes ou plus, totalement ou partiellement, d'abord en isophoronenitrilimine et cette isophoronenitrilimine est hydrogénée avec amination, en tant que substance pure ou en mélange avec d'autres composants et/ou de l'isophoronenitrile, en présence au moins d'ammoniac, d'hydrogène et d'un catalyseur ;
le catalyseur présentant les propriétés suivantes :
I. le catalyseur présente, après activation, dans sa totalité, la composition suivante en pourcentage en poids (% en poids), la somme des proportions valant 100% en poids, par rapport aux métaux contenus :
cobalt : 55 à 85% en poids
aluminium : 5 à 43,5% en poids
chrome : 0,5 à 3% en poids
nickel : 1 à 7% en poids
et
II. le catalyseur se trouve sous forme de particules irrégulières en tant que granulat et présente, après l'activation, des grosseurs de particule de 1 à 8 millimètres (mm), mesurées par tamisage.

2. Procédé de préparation d'isophoronediamine selon la revendication 1, **caractérisé en ce que**
I. le catalyseur présente, après activation, dans sa totalité, la composition suivante en pourcentage en poids (% en poids), la somme des proportions valant 100% en poids, par rapport aux métaux contenus :
cobalt : 57 à 84% en poids
aluminium : 10 à 40% en poids
chrome : 1 à 2% en poids
nickel : 2 à 4% en poids

3. Procédé de préparation d'isophoronediamine selon au moins l'une des revendications précédentes 1-2, **caractérisé en ce que**
les grosseurs de particule du catalyseur, c'est-à-dire les particules de granulat varient de 2,5 à 6 millimètres (mm)
ou
les grosseurs de particule du catalyseur, c'est-à-dire les particules de granulat varient de 3 à 7 millimètres (mm)
ou
les grosseurs de particule du catalyseur, c'est-à-dire les particules de granulat varient de 2 à 5 millimètres (mm) .

4. Procédé de préparation d'isophoronediamine selon au moins l'une des revendications précédentes 1-2, **caractérisé en ce que** le catalyseur est obtenu par tamisage des granulats préparés.

5. Procédé de préparation d'isophoronediamine selon la revendication 4, **caractérisé en ce que** le catalyseur est obtenu par tamisage des granulats préparés et
les grosseurs de particule du catalyseur, c'est-à-dire les particules de granulat se trouvent avec une répartition statistique entre 2,5 à 5,5 millimètres (mm) ou
les grosseurs de particule du catalyseur, c'est-à-dire les particules de granulat se trouvent avec une répartition statistique entre 3,5 à 6,5 millimètres (mm) ou
les grosseurs de particule du catalyseur, c'est-à-dire les particules de granulat se trouvent avec une répartition statistique entre 2 à 5 millimètres (mm) ou les grosseurs de particule du catalyseur, c'est-à-dire les particules de granulat se trouvent avec une répartition statistique entre 3 à 7 millimètres (mm),
et jusqu'à 10 pour cent des particules pouvant se trouver également en dehors de la plage mentionnée de la limite supérieure ou de la limite inférieure mentionnées, mais également à chaque fois jusqu'à 10 pour cent pouvant se trouver en dehors de la plage mentionnée de la limite supérieure et de la limite inférieure mentionnées.

6. Procédé de préparation d'isophoronediamine selon la revendication 1, **caractérisé en ce que** le catalyseur présente, après activation, dans sa totalité, la composition suivante en pourcentage en poids (% en poids), la somme des proportions valant 100% en poids, par rapport aux métaux contenus :
cobalt : 57 à 84% en poids
aluminium : 10 à 40% en poids
chrome : 1 à 2% en poids
nickel : 2 à 4% en poids
et avec
des grosseurs de particule du catalyseur, c'est-à-dire les particules de granulat avec une répartition statistique entre 2,5 à 5,5 millimètres (mm),
ou
des grosseurs de particule du catalyseur, c'est-à-dire les particules de granulat avec une répartition statistique entre 3,5 à 6,5 millimètres (mm),
ou
des grosseurs de particule du catalyseur, c'est-à-dire les particules de granulat avec une répartition statistique entre 2 à 5 millimètres (mm),
ou
des grosseurs de particule du catalyseur, c'est-à-dire les particules de granulat avec une répartition statistique entre 3 à 7 millimètres (mm),
jusqu'à 10 pour cent des particules pouvant se trouver également en dehors de la plage mentionnée de la limite supérieure ou de la limite inférieure mentionnées, mais également à chaque fois jusqu'à 10 pour cent pouvant se trouver en dehors de la plage mentionnée de la limite supérieure et de la limite inférieure mentionnées.

7. Procédé de préparation d'isophoronediamine selon au moins l'une des revendications précédentes, **caractérisé en ce que** les catalyseurs contiennent en plus des métaux dopants, de préférence choisis parmi Mo, Fe, Ag, V, Ga, In, Bi, Ti, Zr et Mn ainsi que les métaux rares.

8. Procédé de préparation d'isophoronediamine selon au moins l'une des revendications précédentes, **caractérisé en ce que** les catalyseurs contiennent des agents de modification, de préférence des métaux alcalins et alcalino-terreux ou leurs composés, de préférence des composés de Mg et de Li.

9. Procédé de préparation d'isophoronediamine selon au moins l'une des revendications précédentes, **caractérisé en ce que**, dans la première étape, au moins une partie de l'isophoronenitrilimine utilisée est convertie en présence ou en l'absence d'un catalyseur d'imination et/ou de solvants, par réaction avec de l'ammoniac en isophoronenitrilimine, la conversion d'IPN en IPNI après l'imination étant supérieure à 80%, de préférence supérieure à 90%, de manière particulièrement préférée supérieure à 95%.

10. Procédé de préparation d'isophoronediamine selon au moins l'une des revendications précédentes, **caractérisé en ce que**, dans la deuxième étape, le produit de réaction de la première étape, tel qu'il est obtenu ou après un traitement ultérieur et/ou ajout d'ammoniac supplémentaire, est hydrogéné avec amination en présence au moins d'ammoniac et d'hydrogène et en présence ou en l'absence d'un solvant organique à une température de 20 à 150°C, de préférence de 40 à 130°C et à une pression de 0,3 à 50 MPa, de préférence de 5 à 30 MPa, sur des catalyseurs d'hydrogénation.

11. Procédé de préparation d'isophoronediamine selon au moins l'une des revendications précédentes, **caractérisé en ce que** la transformation d'IPN en IPDA est effectuée dans trois chambres de réaction séparées les unes des autres, dans lesquelles, dans la première chambre de réaction, la transformation d'IPN en isophoronenitrilimine est effectuée avec de l'ammoniac en excès sur des catalyseurs d'imination à des températures entre 20 et 150°C et à des pressions entre 5 et 30 MPa, dans la deuxième chambre de réaction, les produits de réaction formés sont hydrogénés avec de l'hydrogène en présence d'ammoniac en excès sur des catalyseurs d'hydrogénation à des températures entre 20 et 130°C et à des pressions de 5 à 30 MPa, et dans la troisième chambre de réaction, les produits de réaction formés sont hydrogénés sur des catalyseurs à des températures entre 100 et 160°C et à des pressions de 5 à 30 MPa.

12. Procédé de préparation d'isophoronediamine selon au moins l'une des revendications précédentes, **caractérisé en ce que** la réaction d'imination est effectuée en présence d'au moins un catalyseur d'imination.

13. Procédé de préparation d'isophoronediamine selon au moins l'une des revendications précédentes, **caractérisé en ce que** l'imination d'isophoronenitrile est réalisée avec de l'ammoniac liquide sans ajout d'autres solvants.

14. Procédé de préparation d'isophoronediamine selon au moins l'une des revendications précédentes, **caractérisé en ce qu'**on utilise dans l'étape d'imination, par mole d'IPN utilisé, entre 1 et 500 moles, de préférence entre 5 et 200 moles, de manière particulièrement préférée entre 5 et 100 moles d'ammoniac.

15. Procédé de préparation d'isophoronediamine selon au moins l'une des revendications précédentes, **caractérisé en ce que**
l'imination est réalisée en présence d'un catalyseur en suspension ou d'un catalyseur en lit solide, de préférence d'au moins un catalyseur en lit solide.

16. Procédé de préparation d'isophoronediamine selon au moins l'une des revendications précédentes, **caractérisé en ce que** lors de l'imination, l'IPN et l'ammoniac sont guidés en continu du bas vers le haut à travers un tube de réaction rempli de catalyseur d'imination.

17. Procédé de préparation d'isophoronediamine selon au moins l'une des revendications précédentes, **caractérisé en ce que** l'hydrogène nécessaire pour l'hydrogénation est introduit dans le réacteur soit en excès, de préférence avec jusqu'à 10.000 équivalents en mole, soit en une quantité telle que l'hydrogène consommé par la réaction ainsi que la partie de l'hydrogène qui quitte le réacteur sous forme dissoute dans le flux produit, sont compensés.

18. Procédé de préparation d'isophoronediamine selon au moins l'une des revendications précédentes, **caractérisé en ce que** l'hydrogénation est réalisée dans de l'ammoniac liquide en tant que solvant, en utilisant, par mole d'IPN, entre 1 et 500 moles, de préférence entre 5 et 200 moles, de manière particulièrement préférée entre 5 et 100 moles d'ammoniac.

19. Procédé de préparation d'isophoronediamine selon au moins l'une des revendications précédentes, **caractérisé en ce que** les catalyseurs d'hydrogénation sont d'abord conditionnés avec de l'ammoniac avant leur utilisation dans l'hydrogénation.

20. Procédé de préparation d'isophoronediamine selon au moins l'une des revendications précédentes, **caractérisé en ce que** l'hydrogénation est effectuée en continu dans des réacteurs à lit solide.

21. Procédé de préparation d'isophoronediamine selon au moins l'une des revendications précédentes, **caractérisé en ce que** l'hydrogénation est effectuée en continu dans des réacteurs à lit solide qui fonctionnent selon un mode de lit à ruissellement ou un mode avec un résidu.

22. Procédé de préparation d'isophoronediamine selon au moins l'une des revendications précédentes, **caractérisé en ce que** le mélange réactionnel quittant l'hydrogénation est purifié en une ou plusieurs étapes et l'isophoronediamine est obtenue.

23. Procédé de préparation d'isophoronediamine selon au moins l'une des revendications précédentes, **caractérisé en ce que** le mélange réactionnel quittant l'hydrogénation est purifié en deux étapes, dans lesquelles, dans une première étape, en particulier de l'hydrogène, des gaz inertes, de l'ammoniac, des impuretés à bas point d'ébullition et le cas échéant de l'eau sont séparés totalement ou partiellement dans une ou plusieurs colonnes de distillation et, dans une deuxième étape, d'autres impuretés à bas point d'ébullition, de l'eau et des impuretés à point d'ébullition élevé sont séparés totalement ou partiellement dans des colonnes de distillation et l'isophoronediamine est obtenue.

24. Utilisation d'un catalyseur pour la préparation d'isophoronediamine, **caractérisée en ce que**
A) de l'isophoronenitrile est hydrogéné avec amination, directement en une étape en présence d'ammoniac, d'hydrogène, d'un catalyseur d'hydrogénation et le cas échéant d'autres additifs et en présence ou en l'absence de solvants organiques en isophoronediamine ; ou
B) de l'isophoronenitrile est converti en au moins deux étapes ou plus, totalement ou partiellement, d'abord en isophoronenitrilimine et cette isophoronenitrilimine est hydrogénée avec amination, en tant que substance pure ou en mélange avec d'autres composants et/ou de l'isophoronenitrile, en présence au moins d'ammoniac, d'hydrogène et d'un catalyseur ;
le catalyseur présentant les propriétés suivantes :
I. le catalyseur présente, après activation, dans sa totalité, la composition suivante en pourcentage en poids (% en poids), la somme des proportions valant 100% en poids, par rapport aux métaux contenus :
cobalt : 55 à 85% en poids
aluminium : 5 à 43,5% en poids
chrome : 0,5 à 3% en poids
nickel : 1 à 7% en poids
et
II. le catalyseur se trouve sous forme de particules irrégulières en tant que granulat et présente, après l'activation, des grosseurs de particule de 1 à 8 millimètres (mm), mesurées par tamisage.
